(19) Europäisches Patentamt
European Patent Office
Office européen des brevets

(11) EP 0 810 197 B1

(12) **FASCICULE DE BREVET EUROPEEN**

(45) Date de publication et mention de la délivrance du brevet:
**21.03.2001 Bulletin 2001/12**

(51) Int Cl.[7]: **C07C 67/00**, C07C 69/63

(21) Numéro de dépôt: **97401112.4**

(22) Date de dépôt: **21.05.1997**

(54) **Procédé de préparation d'halogénodifluoroacétates d'alkyle**

Verfahren zum Herstellen von Alkylhalogendifluoracetaten

Process for the preparation of alkyl halogenodifluoroacetates

(84) Etats contractants désignés:
**AT BE CH DE DK ES FI FR GB GR IE IT LI LU NL PT SE**

(30) Priorité: **29.05.1996 FR 9606602**

(43) Date de publication de la demande:
**03.12.1997 Bulletin 1997/49**

(73) Titulaire: **Atofina**
**92800 Puteaux (FR)**

(72) Inventeurs:
- **Gillet, Jean-Philippe**
  **69530 Brignais (FR)**
- **Ruppin, Christophe**
  **69310 Pierre-Benite (FR)**

(56) Documents cités:
**EP-A- 0 716 074**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 810 197 B1

Printed by Jouve, 75001 PARIS (FR)

## Description

La présente invention concerne un procédé de préparation directe d'halogénodifluoroacétates d'alkyle par réaction de 1,1-difluorotétrahalogènoéthanes sur un alcool.

Les halogénodifluoroacétates d'alkyle sont des intermédiaires de synthèse de produits pharmaceutiques et phytosanitaires.

De nombreuses méthodes ont été décrites pour obtenir ces halogénodifluoroacétates d'alkyle.

Le plus souvent, elles font appel à la réaction d'un alcool sur un acide halogénodifluoroacétique, ou de préférence sur les fluorures et les chlorures correspondants.

Ces halogénures (fluorure ou chlorure) d'halogénodifluoroacétyle peuvent être obtenus selon des techniques très variées.

Le brevet US 5259 938 décrit un procédé de préparation de chlorure d'ω-halogénodifluoroacétyle $M(CF_2)_nCOCl$, avec M = F ou Cl et n allant de 1 à 10, par oxydation photochimique en présence de chlore de composés de formule $M(CF_2)_n\ CH_xCl_y$ avec x = 1 ou 2 sachant que x + y = 3.

Dans le Journal of Organic Chemistry, 33 (2) p. 816-9 (1968) on décrit un procédé d'accès au chlorure de bromodifluoroacétyle qui comporte les étapes suivantes :

$$CF_2 = CF_2 + NaOCH_3 \xrightarrow{THF} CF_2 = C(F)(OCH_3) \xrightarrow{Brome}$$

$$\longrightarrow CF_2BrCFBrOCH_3$$

$$CF_2BrCFBrOCH_3 + 2ClSO_3H \longrightarrow CF_2BrC(O)Cl$$

Le rendement final en $CF_2BrC(O)Cl$ par rapport au tétrafluoroéthylène $C_2F_4$ est inférieur à 30 %.

Les fluorures de difluorohalogènoacétyles peuvent être obtenus également à partir de $C_2F_4$.

Notamment, le fluorure de bromodifluoroacétyle peut être obtenu selon les étapes ci-après décrites dans la demande de brevet japonais publiée sous le n° JP 82 40434 :

$CF_2BrCF_2Br$ (obtenu selon $CF_2 = CF_2 + Br_2$) + $SO_3$ (ou $HSO_3F$) donne un intermédiaire contenant le groupement $BrCF_2CF_2OSO_2$—.

Cet intermédiaire est chauffé avec $H_2SO_4$ ou KF/sulfolane et conduit au fluorure de bromodifluoroacétyle $CF_2BrC(O)F$.

Les méthodes les plus citées consistent cependant à réaliser une hydrolyse sulfurique, en présence de sels mercuriques, de 1,1-difluorotétrahalogènoéthanes tels que : $CF_2BrCFClBr$, $CF_2ClCCl_3$.

Ainsi MOREL D. & DAWANS F. (Tetrahedron, 33 (12) pp1445-7) mentionnent que le 1,2-dibromochlorotrifluoroéthane obtenu par bromation du chlorotrifluoroéthylène est hydrolysé en milieu oléum en présence d'HgO selon la réaction :

$$CF_2BrCFClBr \xrightarrow[HgO]{oléum} CF_2\ BrC(O)F$$

Avec un oléum à 30-40 %, la quantité de HgO utilisée pour activer la réaction est de l'ordre de 1 % en poids par rapport à $CF_2BrCFClBr$. Si la concentration de l'oléum est supérieure à 60 %, l'oxyde de mercure peut être supprimé.

Le brevet DE 1020970 décrit la préparation de $CF_2ClC(O)Cl$ selon une méthode analogue qui consiste à traiter $CF_2ClCClBr_2$ avec un oléum en présence de $HgSO_4$ à une température voisine de 50° C selon la réaction :

$$CF_2ClCClBr_2 + \text{oléum } (65\ \%\ SO_3) \xrightarrow[50\ -\ 60°\ C]{HgSO4} SO_2 + CF_2ClC(O)Cl$$

Le $CF_2ClC(O)Cl$ peut être purifié par chloration catalytique en phase gazeuse puis séparé par distillation fractionnée.

Notons également que l'on peut partir des oxydes d'oléfines perhalogénés tels que l'oxyde de tétrafluoroéthylène ou

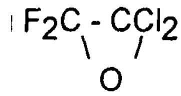

Le brevet EP 380 129 décrit la préparation de $CF_2BrC(O)F$ selon la réaction :

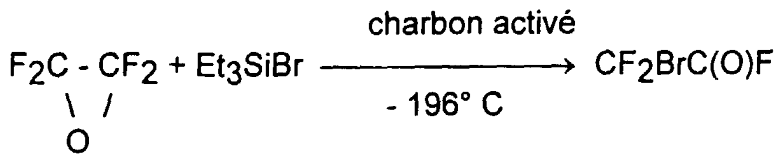


Signalons enfin que Chang-Ming Hu et Coll. (Journal of Fluorine Chemistry, 49 (1990) p. 275-280) ont décrit une méthode assez générale qui convertit un halogénoéthane en acide correspondant en faisant réagir des quantités stoechiométriques de polyfluoroperhalogénoalcane et d'un couple redox constitué de persulfate d'ammonium et de formiate de sodium.

Ainsi le 1,1-difluorotetrachloroéthane est converti en acide chlorodifluoroacétique selon la réaction :

$$CF_2ClCCl_3 + (NH_4)_2\ S_2O_8 + HCO_2Na,\ 2H_2O \rightarrow CF_2ClCOOH$$

(rendement 66,5 % - conversion 100 %)

La réaction s'effectue à 30° C en milieu DMF avec un bullage d'air. La réaction terminée, le milieu est versé dans de l'eau et la solution fortement acide est extraite avec de l'éther. L'extrait à l'éther est neutralisé avec une solution aqueuse de $NaHCO_3$. La phase aqueuse est évaporée à sec puis le résidu ($CF_2ClCO_2Na$) est repris avec $H_2SO_4$ concentré puis distillé.

Toutes ces méthodes présentent de nombreux inconvénients. Elles utilisent le plus souvent des milieux réactionnels corrosifs (oléum - $H_2SO_4$ concentré), des catalyseurs dangereux pour l'environnement (sels de mercure) ou bien font appel à des réactions susceptibles de dégager des gaz corrosifs tels que HF. Ceci entraîne, d'une part, des appareillages spécifiques et coûteux (revêtement en PVDF ou PTFE) et d'autre part, des traitements complexes des effluents si l'on veut préserver l'environnement.

Par ailleurs, il y a lieu de noter que certaines matières premières sont d'accès difficiles ou bien de manipulation dangereuse nécessitant des appareillages très spécifiques.

Le procédé qui fait l'objet de la présente invention permet d'obtenir directement de façon simple avec des rendements élevés, à partir de réactifs aisément accessibles, les halogénodifluoroacétates d'alkyle de formule :

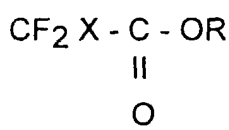 (I)

dans laquelle X représente un atome de fluor, de chlore, de brome ou d'iode,

R représente un radical hydrocarboné aliphatique, linéaire ou ramifié ayant un nombre de carbone allant de 1 à 10, et de préférence, allant de 1 à 8, ce procédé étant caractérisé en ce qu'il comprend :

1°/ à mettre en contact un 1,1-difluorotétrahalogénoéthane de formule :

$$CF_2XCY_2Z \qquad (II)$$

dans laquelle X a la même signification que dans la formule (I), Y et Z, identiques ou différents représentent

un atome de brome, de chlore ou d'iode, avec un alcool ROH (III), R ayant la même signification que dans la formule (I), selon un rapport molaire $ROH/CF_2\ XCY_2Z$ au plus égal à 30 et, de préférence compris entre 5 et 20, en présence d'oxygène et d'une quantité molaire mineure p étant au plus égale à 40% de la quantité molaire totale p + q mise en oeuvre d'au moins un initiateur chimique de radicaux libres ;

2°/ à chauffer le milieu réactionnel obtenu dans l'étape 1°/ à une température au moins égale à 40° C et, de préférence, comprise entre 60° C et 80° C ;

3°/ à introduire en continu ou par ajouts successifs, tout en maintenant la température de l'étape 2°/ et toujours en présence d'oxygène, une quantité molaire majeure q d'au moins un initiateur chimique de radicaux libres tel que le rapport molaire $p+q/CF_2XCY_2Z$ soit compris entre 0,01 et 0,2 et, de préférence, compris entre 0,05 et 0,1 ;

4°/ à refroidir le milieu réactionnel à température ambiante puis à extraire par distillation dudit milieu réactionnel un produit brut puis à soumettre cedit produit brut à une distillation en présence d'un solvant aliphatique, cycloaliphatique ou aromatique ;

5°/ à récupérer l'halogénodifluoroacétate d'alkyle (I).

Par produit brut, on désigné selon la présente invention un mélange comprenant de l'eau, ROH en excès, l'halogénodifluoroacétate d'alkyle (I), le 1,1-difluorotétrahalogénoéthane (II) non transformé et divers sous produits. Ce produit brut représente au moins 60 % et de préférence 70 % en poids du milieu réactionnel.

A titre d'illustration de 1,1-difluorotétrahalogènoéthanes (II) utilisables selon la présente invention, on peut citer les composés de formule :

$$CF_2BrCCl_2Br,\ CF_2ClCCl_2I,\ CF_2BrCCl_2I,\ CF_3CBr_3.$$

Tous ces composés sont obtenus de manière connue et ne font pas l'objet de la présente invention.

On ne sortirait pas du cadre de l'invention si l'on opérait l'étape 1°/ en présence d'eau. Cette quantité pondérale d'eau peut varier dans une large mesure et peut atteindre 50 % de la masse totale des réactifs.

A titre d'illustration d'alcool ROH utilisables selon la présente invention on peut citer le méthanol, l'éthanol, l'isopropanol, le propanol, le n-butanol, le 2-éthyl hexanol.

A titre d'illustration d'initiateur chimique de radicaux libres utilisable selon la présente invention, on peut citer le peroxyde d'hydrogène, les peroxydes organiques comme le peroxyde de benzoyle, le peroxyde de lauroyle, le peroxyde d'acétylcyclohexanesulfonyle, le peroxyde d'isobutyroyle, le peroxyde de dichloracétyle, le peroxyde de trichloracétyle ; les hydroperoxydes organiques comme l'hydroperoxyde de tertiobutyle, l'hydroperoxyde de tertioamyle, l'hydroperoxyde de cumène, l'hydroperoxyde de paramenthane ; les peroxydicarbonates comme le peroxydicarbonate d'éthyle, le peroxydicarbonate d'éthylhexyle, le peroxydicarbonate d'isopropyle, le peroxydicarbonate d'isobutyle, le peroxydicarbonate de cétyle, le peroxydicarbonate de cyclohexyle, le peroxydicarbonate de myristyle, le peroxydicarbonate de tertiobutylcyclohexyle ; le pernéodécanoate de tertiobutyle, le pernéodécanoate de cumyle ; le perméthoxyacétate de tertiobutyle ; le peréthoxyacétate de tertiobutyle ; les composés azoïques comme le 2,2'-azo-bis (2,4-diméthylvaléronitrile), le 1,1-azobis (cyclohexane carbonitrile), l'azobis (isobutyronitrile), le 2,2'azobis(2-méthylbutyronitrile).

Parmi ces initiateurs chimiques on préfère utiliser les composés azoïques tels que l'azobis(isobutyronitrile) désigné par AIBN ou le 2,2'azobis(2-méthylbutylronitrile) désigné par AMBN. On préfère utiliser tout particulièrement l'AMBN.

Selon la présente invention, on peut utiliser dans les étapes 1°/ et 2°/ des initiateurs chimiques de radicaux libres identiques ou différents mais, de préférence, on utilisera un initiateur identique.

La quantité molaire mineure p d'initiateur chimique de radicaux libres est, de préférence, comprise entre 10 % et 20 % de la quantité molaire totale p + q mise en oeuvre dans les étapes 1°/ et 3°/ du procédé.

Comme solvant susceptible de former un azéotrope avec ROH et/ou l'eau on peut utiliser tout solvant non alcoolique qui ne doit ni être réactif à l'égard des réactifs (II) et (III) ni avoir d'influence sur les produits formés (I).

Il doit être également totalement inerte dans les conditions opératoires de distillation. A titre d'illustration de tels solvants utilisables selon la présente invention, on peut citer les paraffines linéaires tels que l'hexane, l'heptane, les cycloparaffines tels que le cyclohexane, le cycloheptane, les aromatiques tels que le toluène, le benzène, le cumène, les xylènes.

Selon la présente invention, on utilisera une quantité pondérale de solvant comprise entre 50 % et 200 % et, de préférence, une quantité comprise entre 60 % et 100 % par rapport au produit brut.

Selon une variante du procédé on peut opérer en ajoutant dans l'étape 1°/ un solvant identique ou différent de celui utilisé à l'étape 4°/. Dans cette variante, on supprime l'opération d'extraction par distillation du produit brut du milieu réactionnel.

Selon la présente invention, la durée de l'étape 3°/ peut varier dans une large mesure. Elle est au moins

égale à 1 heure et, de préférence, comprise entre 6 heures et 24 heures.

Selon la présente invention, la réaction s'effectue en présence d'oxygène ou d'air, ou bien d'air enrichi en oxygène, ou bien encore d'un gaz inerte tel que l'azote ou l'argon enrichi en oxygène. Selon la présente invention, on opérera avec une quantité molaire d'oxygène au moins égale à 1 mole par mole de (II) mis en oeuvre.

On opère généralement à pression atmosphérique ($10^5$ Pa) mais on ne sortirait pas du cadre de l'invention si on opérait à une pression différente.

L'invention s'applique tout particulièrement à la préparation du bromodifluoroacétate d'éthyle, du chlorodifluoroacétate d'éthyle et du trifluoroacétate d'éthyle.

D'une manière générale, les produits de formule (I) sont préparés dans tout dispositif permettant une bonne dispersion de l'oxygène dans le milieu réactionnel.

On opérera tout particulièrement dans un réacteur cylindrique vertical (en verre) muni d'une double enveloppe dans laquelle peut circuler un fluide caloporteur ou de refroidissement, équipé d'un réfrigérant ascendant en liaison avec une garde hydraulique, d'un tube plongeur, d'une prise de température, d'une introduction et dont la base est constituée par une plaque poreuse par laquelle l'oxygène est diffusé dans le réacteur.

La porosité de la plaque située à la base dudit réacteur est telle qu'elle permet d'obtenir une bonne diffusion de l'oxygène dans le milieu réactionnel.

Ledit réacteur peut éventuellement être muni d'un agitateur ou bien encore d'une boucle de recirculation.

On prépare les produits de formule (I) avantageusement selon le mode opératoire ci-après. On introduit dans un réacteur comme décrit précédemment et sous courant d'oxygène (ledit oxygène arrivant à la base dudit réacteur et traversant la plaque poreuse située à la base du réacteur)le 1,1-difluorotetrahagénoéthane (II), l'alcool ROH (III), une quantité p d'initiateur chimique de radicaux libres et éventuellement de l'eau.

On chauffe le milieu réactionnel toujours sous courant d'oxygène.

On introduit ensuite en continu ou par ajouts successifs la quantité q d'initiateur chimique de radicaux libres.

L'addition terminée, on refroidit le milieu réactionnel puis on effectue une première distillation permettant d'extraire du milieu réactionnel un produit brut tel que défini précédemment. Ensuite, on soumet ce produit brut à une distillation en présence d'un solvant susceptible de former un ou plusieurs azéotropes (binaire et/ou ternaire) avec l'eau et/ou l'alcool. Ensuite, le résidu de cette distillation est soumis à une purification pour récupérer l'halogénodifluoroacétate d'alkyle (I). Cette purification consiste le plus souvent à effectuer une distillation sous pression réduite.

Les réactifs non-transformés, tels que notamment l'alcool ROH, et le solvant peuvent être recyclés.

Les produits sont analysés par chromotographie en phase gazeuse et identifiés par résonance magnétique nucléaire.

Le procédé qui fait l'objet de la présente invention permet d'obtenir des halogénodifluoroacétates d'alkyle (I) directement, selon des conditions opératoires douces par simple réaction entre un 1,1-difluorotétrahalogénoéthane et un alcool avec une conversion élevée du 1,1-difluorotétrahalogénoéthane en ester. En outre, les effluents sont constitués de produits qui peuvent être éventuellement recyclés, tel que notamment le solvant.

Les exemples qui suivent illustrent l'invention.

**EXEMPLE 1**

Dans un réacteur tubulaire de 430 ml, muni d'une double enveloppe dans laquelle circule un fluide caloporteur, d'un disperseur de gaz à sa base (verre fritté), d'une arrivée de gaz située à la partie inférieure dudit réacteur, d'un condenseur en liaison avec un piège refroidi, d'une ampoule de coulée, d'une sonde de température, d'un tube d'alimentation, on injecte un courant d'air à un débit de 8l/h au travers du disperseur puis on introduit successivement :

- 74,03 g (soit 0,253 mole) de $CF_2BrCl_2Br$,
- 174,5 g (soit 3,789 moles) d'éthanol ce qui correspond à un rapport molaire éthanol/$CF_2BrCCl_2Br$ égale à environ 15, et
- 486 mg de 2,2'-azobis(2-méthyl butyronitrile), ci-après désigné par AMBN, comme initiateur chimique de radicaux libres.

L'ensemble est chauffé à 70° C au moyen d'une huile circulant dans la double enveloppe.

Le débit d'air de 8l/h et la température de 70° C sont maintenus pendant 12 heures temps pendant lequel on ajoute toutes les deux heures 486 mg d'AMBN, soit 5 x 486 = 2,430 g d'AMBN (0,0126 mole). Ceci correspond à un rapport molaire initiateur/$CF_2BrCCl_2Br$ égal à :

$$\frac{0{,}0025 + 0{,}0126}{0{,}253} = 0{,}0596$$

L'avancement de la réaction est effectué en déterminant la conversion de $CF_2BrCCl_2Br$ par analyse en chromatographie en phase gazeuse (CPG) de prélèvements effectués sur le milieu réactionnel. Les résultats sont reportés ci-après :

| *Temps de réaction (heure)* | *Conversion de $CF_2BrCCl_2Br$ (%)* |
|---|---|
| 1 | 24 |
| 5 | 65 |
| 12 | 91 |

On refroidit le milieu réactionnel à température ambiante. On obtient 236,4 g d'un mélange que l'on soumet à une distillation dans une colonne adiabatique type "Oldershaw" de 20 plateaux munie d'un timer.

On porte la température du pied de la colonne jusqu'à 88° C, on laisse la colonne s'équilibrer et on soutire un produit brut en tête de colonne avec un reflux de 9/1, jusqu'à une température de 77,5° C. Après 8h50min de distillation, on stoppe la distillation.

On obtient 165,5 g d'un produit brut (environ 70 % en poids du mélange réactionnel soumis à la distillation) qui comprend de l'éthanol (40 % en poids) du bromodifluoroacétate d'éthyle (19,57 % en poids), de l'eau,...

Ce produit brut est soumis à une seconde distillation en présence de 100 g de cyclohexane afin d'éliminer l'eau et l'éthanol.

On utilise la même colonne que précédemment.

La tête de colonne est munie d'un Timer et d'un Florentin pour le recyclage de la phase organique (cyclohexane).

On porte la température du pied de la colonne à 90° C, on laisse la colonne s'équilibrer. On distille en tête de colonne, à une température de 62,1° C l'azéotrope ternaire eau, éthanol, cyclohexane, puis après, on distille à 64,9° C l'azéotrope binaire éthanol/cyclohexane avec un reflux de 9/1.

On distille ensuite l'excès de cyclohexane.

Le bromodifluoroacétate d'éthyle (BDFAE) obtenu en pied de colonne de pureté d'environ 95 % est soumis à une nouvelle distillation sous pression réduite.

On obtient 34 g de bromodifluoroacétate d'éthyle distillant à 57° C/58° C sous une pression de 133,32 Pa (100 mm Hg)..

La pureté déterminée par CPG est de 98,5 %.

Le rendement molaire en bromodifluoroacétate d'éthyle obtenu (pureté : 98,5 %) est de 68 % par rapport au $CF_2BrCCl_2Br$ mis en oeuvre.

Le bromodifluoroacétate d'éthyle a été identifié par résonance magnétique nucléaire (RMN) du proton ($H^1$), du carbone 13 ($C^{13}$) et du fluor 19 ($F^{19}$) sur un appareil Brücker type AC300 multinoyaux (fréquences pour $H^1$ = 300,13 MHz, pour $C^{13}$ = 75,47 MHz et pour $F^{19}$ = 282,4 MHz).

Identification RMN de

a b c d
$CF_2BrCOCH_2CH_3$
||
O

- Spectre RMN du $C^{13}$
  (solvant = $CDCl_3$)

  $\delta a$ = 108,8 ppm
  $\delta b$ = 159,5 ppm
  $\delta c$ = 64,5 ppm
  $\delta d$ = 13,5 ppm

- Spectre RMN du $F^{19}$
  (solvant = $CDCl_3$/étalon externe : acide trifluoroacétique)

$\delta(CF_2Br)$ = - 16,8 ppm
constante de couplage $J_{C\text{-}F}$= 314 Hz
constante de couplage $J_{C\text{-}F}$ = 31 Hz

- ♦ Spectre RMN du $H^1$
  (solvant = $CDCl_3$/étalon interne : tétraméthylsilane)

  $\delta(CH_2)$ = 4,42 ppm
  $\delta(CH_3)$ = 1,40 ppm

**EXEMPLE 2**

On opère dans le même appareillage que l'exemple 1.
On injecte un courant d'air à un débit de 8l/h au travers du disperseur, puis on introduit successivement :

- 38 g (soit 0,130 mole) de $CF_2BrCCl_2Br$,
- 87,2 g (soit 1,893 moles) d'éthanol, ce qui correspond à un rapport molaire éthanol/$CF_2BrCCl_2Br$ égale à 14,56, et 242,5 mg (soit 0,0013 mole) d'AMBN.

L'ensemble est chauffé à 70° C au moyen d'une huile circulant dans la double enveloppe.
Le débit d'air de 8l/h et la température de 70° C sont maintenus pendant 12 heures, temps durant lequel on injecte en continu 15,15 g d'une solution mère éthanolique d'AMBN à raison de 1,262 g/h.
Cette solution est constituée par 1,248g (soit 0,0065 mole) d'AMBN, 9,7 g d'éthanol et 4,2 g (soit 0,143 mole) de $CF_2BrCCl_2Br$.
Ceci correspond à un rapport molaire initiateur/$CF_2BrCCl_2Br$ égale à :

$$\frac{0{,}0013 + 0{,}0065}{0{,}130 + 0{,}014} = 0{,}0542$$

Comme dans l'exemple 1, l'avancement de la réaction est effectué en déterminant la conversion de $CF_2BrCCl_2Br$ par analyse CPG de prélèvements effectués sur le milieu réactionnel.
Les résultats sont reportés ci-après :

| *Temps de réaction (heure)* | *Conversion de $CF_2BrCCl_2Br$ (%)* |
|---|---|
| 1 | 18 |
| 5 | 45 |
| 12 | 72 |

On refroidit le milieu réactionnel à température ambiante.
On obtient 131,5 g d'un mélange qui est traité selon les conditions de distillation de l'exemple 1.
Le produit brut obtenu après la première distillation (93 g) est soumis à une seconde distillation en présence de 60 g de cyclohexane.
Le bromodifluoroacétate d'éthyle obtenu après distillation sous pression réduite a une pureté identique à celui obtenu dans l'exemple 1.
On obtient 14,25 g de bromodifluoroacétate d'éthyle ce qui correspond à un rendement molaire de 54 % par rapport au $CF_2BrCCl_2Br$ total mis en oeuvre.

**EXEMPLE 3**

On opère dans le même appareillage que l'exemple 1 et selon des conditions opératoires identiques excepté que l'on utilise de l'éthanol à 95 %.
> quantité de réactifs mis en oeuvre :

- 37,12 g (soit 0,127 mole) de $CF_2BrCCl_2Br$,
- Alcool à 95 % 93,21 g ce qui correspond à 88,55 g d'éthanol 100 % soit 1,922 moles et 4,66 g d'eau,
- 242,5 mg (soit 0,0013 mole) d'AMBN.

Le débit d'air est de 81/h, la température est de 70° C et la durée de réaction est de 12 heures temps durant lequel on ajoute toutes les deux heures 242,5 mg d'AMBN.

Ceci correspond à un rapport molaire initiateur/$CF_2BrCCl_2Br$ égal à

$$\frac{0,0013 + 5 \times 0,0013}{0,127} = 0,0614$$

L'avancement de la réaction est effectuée en déterminant la conversion de $CF_2BrCCl_2Br$ comme dans les exemples précédents.

Les résultats sont reportés ci-après :

| *Temps de réaction (heure)* | *Conversion de $CF_2BrCCl_2Br$ (%)* |
|---|---|
| 1 | 35 |
| 3 | 48 |
| 5 | 65 |
| 7 | 79 |
| 9 | 87 |
| 12 | 99 |

On refroidit le milieu réactionnel à température ambiante.

On obtient 119 g d'un mélange qui est traité selon les conditions de distillation de l'exemple 1.

Le produit brut obtenu après la première distillation (environ 85 g) est soumis à une seconde distillation en présence de 70 g de cyclohexane.

On obtient après distillation sous pression réduite 15,2 g de bromodifluoroacétate d'éthyle de pureté égale à environ 98 % ce qui correspond à un rendement molaire de 59 % par rapport au $CF_2BrCCl_2Br$ mis en oeuvre.

**EXEMPLE 4**

On opère dans le même appareillage que l'exemple 1. On injecte un courant d'air à un débit de 81/h au travers du disperseur, puis on introduit successivement :

- 37,21 g (soit 0,127 mole) de $CF_2BrCCl_2Br$,
- 114,08 g (soit 1,90 mole) d'isopropanol, ce qui correspond à un rapport molaire isopropanol/$CF_2BrCCl_2Br$ de 14,96 ; et
- 244,6 mg (soit 0,00127 mole) d'AMBN.

L'ensemble est chauffé à 70°C au moyen d'une huile circulant dans la double enveloppe.

Le débit d'air de 8l/h et la température de 70°C sont maintenus pendant 7 heures, temps durant lequel on ajoute toutes les deux heures 242,6 mg d'AMBN soit 3 x 242,6 = 727,8 mg d'AMBN (0,00379 mole). Ceci correspond à un rapport molaire initiateur/$CF_2BrCCl_2Br$ égal à

$$\frac{0,00127 + 0,00379}{0,127} = 0,0398$$

Comme dans l'exemple 1, l'avancement de la réaction est effectué en déterminant la conversion de $CF_2BrCCl_2Br$ par analyse CPG de prélèvements effectués sur le milieu réactionnel.

Les résultats sont reprotés ci-après :

| *Temps de réaction (heure)* | *Conversion de $CF_2BrCCl_2Br$ (%)* |
|---|---|
| 1 | 48 |
| 3 | 70 |
| 5 | 90 |
| 7 | 98 |

On refroidit le milieu réactionnel à température ambiante.
On obtient 142,3 g d'un mélange qui est traité selon les conditions de distillation de l'exemple 1. Le produit brut obtenu après ia première distillation (99,6 g) est soumis à une seconde distillation en présence de 70g de toluène.
Le bromodifluoroacétate d'isopropyle obtenu après distillation sous pression réduite à une pureté supérieure à 99 %.
On obtient 19,7 g de bromodifluoroacétate d'isopropyle, ce qui correspond à un rendement molaire de 71 % par rapport au $CF_2BrCCl_2Br$ mis en oeuvre.

**EXEMPLE 5**

On opère dans le même appareillage que dans l'exemple 1. On injecte un courant d'air à un débit de 16l/h au travers du disperseur, puis on introduit successivement :

- 35,31 g (soit 0,12 mole) de $CF_2BrCCl_2I$,
- 82,84 g (soit 1,8 moles) d'éthanol, ce qui correspond à un rapport molaire éthanol/$CF_2BrCCl_2I$ de 15 ; et
- 0,1157 mg (soit 0,0006 mole) d'AMBN.

L'ensemble est chauffé à 71°C.
Le débit d'air de 16l/h et la température de 71°C sont maintenus pendant 2 heures, temps durant lequel on ajoute toutes les 30 minutes 0,1157 g d'AMBN soit 3 x 0,1157 = 0,3471 g d'AMBN (0,0018 mole). Ceci correspond à un rapport molaire initiateur/$CF_2BrCCl_2I$ égal à

$$\frac{0,0006 + 0,0018}{0,1195} = 0,020$$

La conversion de $CF_2BrCCl_2I$ au bout de 2 heures est de 81 %.
On refroidit le milieu réactionnel à température ambiante.
On obtient 117,1 g d'un mélange qui est traité selon les conditions de distillation de l'exemple 1.
Le produit brut obtenu après la première distillation (82 g) est soumis à une seconde distillation en présence de 60 g de cyclohexane.
Le chlorodifluoroacétate d'éthyle obtenu après distillation sous pression réduite a une pureté d'environ 99 % (déterminée par CPG).
Le rendement molaire en chlorodifluoroacétate d'éthyle obtenu (pureté : 99 %) est de 65 % par rapport au $CF_2BrCCl_2I$ mis en oeuvre.
Le chlorodifluoroacétate d'éthyle a été identifié par RMN du $C^{13}$, $F^{19}$ et $H^1$.
Identification RMN du

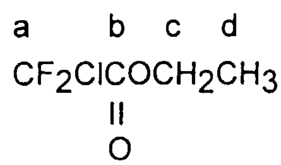

- Spectre RMN du $C^{13}$
  (solvant = $CDCl_3$)

  $\delta a$ = 116,9 ppm
  $\delta b$ = 159,2 ppm
  $\delta c$ = 64,5 ppm
  $\delta d$ = 13,7 ppm

- Spectre RMN du $F^{19}$
  (solvant = $CDCl_3$/étalon externe TFA)

  $\delta$ ($CF_2Cl$) = - 15,4 ppm
  $J1_{C-F}$ = 300 Hz, $J2_{C-F}$ = 34,5 Hz

- Spectre RMN du H[1]
  (solvant = $CDCl_3$/étalon interne TMS)

  δ ($C\underline{H}_2$) = 4,4 ppm
  δ ($C\underline{H}_3$) = 1,4 ppm

**Revendications**

1. Procédé de préparation d'halogénodifluoroacétates d'alkyle de formule :

$$CF_2X - \underset{\underset{O}{\|}}{C} - OR \qquad (I)$$

dans laquelle X représente un atome de fluor, de chlore, de brome ou d'iode, R représente un radical hydrocarboné aliphatique, linéaire ou ramifié ayant un nombre de carbone allant de 1 à 10, caractérisé en ce qu'il comprend :

1°/ à mettre en contact un 1,1-difluorotétrahalogénoéthane de formule :

$$CF_2\ XCY_2\ Z \qquad (II)$$

dans laquelle X a la même signification que dans la formule (I), Y et Z, identiques ou différente représentent un atome de brome, de chlore ou d'iode, avec un alcool ROH (III), R ayant la même signification que dans la formule (I), selon un rapport molaire ROH/$CF_2$ $XCY_2Z$ au plus égal à 30 et, de préférence compris entre 5 et 20 en présence d'oxygène et d'une quantité molaire mineure p étant au plus égale à 40% de la quantité molaire totale p + q mise en oeuvre d'au moins un initiateur chimique de radicaux libres ; d'au moins un initiateur chimique de radicaux
2°/ à chauffer le milieu réactionnel obtenu dans l'étape 1°/ à une température au moins égale à 40° C et, de préférence, comprise entre 60° C et 80° C ;
3°/ à introduire en continu ou par ajouts successifs, tout en maintenant la température de l'étape 2°/ et toujours en présence d'oxygène, une quantité molaire majeure q d'au moins un initiateur chimique de radicaux libres tel que le rapport molaire p + q/$CF_2XCY_2Z$ soit compris entre 0,01 et 0,2 et, de préférence, compris entre 0,05 et 0,1.
4°/ à refroidir le milieu réactionnel à température ambiante puis à extraire par distillation dudit milieu réactionnel un produit brut puis à soumettre cedit produit brut à une distillation en présence d'un solvant aliphatique, cycloaliphatique ou aromatique ;
5°/ à récupérer l'halogénodifluoroacétate d'alkyle (I).

2. Procédé selon la revendication 1, caractérisé en ce que le 1,1-difluorotétrahalogénoéthane de formule (II) est $CF_2BrCCl_2Br$ ou $CF_2BrCCl_2I$.

3. Procédé selon la revendication 1, caractérisé en ce que l'alcool ROH (III) est l'éthanol ou l'isopropanol.

4. Procédé selon l'une des revendications 1 à 3, caractérisé en ce que l'initiateur chimique de radicaux libres est un composé azoïque.

5. Procédé selon la revendication 4, caractérisé en ce que le composé azoique est le 2,2'azobis(2-méthylbutyronitrile).

6. Procédé selon la revendication 1, caractérisé en ce que la quantité molaire mineure p d'initiateur chimique de radicaux libres est comprise entre 10 % et 20 % de la quantité molaire totale p + q mise en oeuvre.

7. Procédé selon la revendication 1, caractérisé en ce que le solvant est le cyclohexane ou le toluène.

**8.** Procédé selon l'une des revendications 1 à 7, caractérisé en ce que l'on opère en présence d'air.

**9.** Procédé selon la revendication 1, caractérisé en ce que l'on opère en présence d'eau.

## Patentansprüche

**1.** Verfahren zur Herstellung von Alkylhalogendifluoracetaten der Formel:

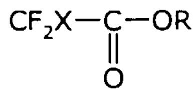

(I)

in der X ein Fluor-, Chlor-, Brom- oder Iodatom und R einen aliphatischen, linearen oder verzweigten Kohlenwasserstoffrest mit 1 bis 10 Kohlenstoffatomen bedeutet, dadurch gekennzeichnet, daß es umfaßt:

1 ) ein 1,1-Difluortetrahalogenethan der Formel:

$$CF_2XCY_2Z \qquad (II)$$

in der X dieselbe Bedeutung wie in Formel (I) hat und die gleichen oder verschiedenen Y und Z ein Brom-, Chlor- oder Iodatom bedeuten, mit einem Alkohol ROH (III), wobei R dieselbe Bedeutung wie in Formel (I) hat, mit einem Molverhältnis ROH/$CF_2XCY_2Z$ von höchstens 30, vorzugsweise zwischen 5 und 20, in Gegenwart von Sauerstoff und einer geringen molaren Menge p mindestens eines chemischen Freie-Radikale-Bildners, die höchstens 40 % der gesamten eingesetzten molaren Menge p+q ist, zusammenzubringen;
2 ) das in Schritt 1) erhaltene Reaktionsmilieu auf eine Temperatur von mindestens 40 °C und vorzugsweise zwischen 60 °C und 80 °C zu erwärmen;
3 ) kontinuierlich oder durch aufeinanderfolgende Zugaben, während die Temperatur von Schritt 2) gehalten wird und immer in Gegenwart von Sauerstoff, eine große molare Menge q mindestens eines chemischen Freie-Radikale-Bildners zuzugeben, so daß das Molverhältnis p+q/ $CF_2XCY_2Z$ zwischen 0,01 und 0,2 und vorzugsweise zwischen 0,05 und 0,1 liegt;
4 ) das Reaktionsmilieu auf Umgebungstemperatur abzukühlen und dann durch Destillation des genannten Reaktionsmilieus ein Rohprodukt abzutrennen und dann dieses Rohprodukt einer Destillation in Gegenwart eines aliphatischen, cycloaliphatischen oder aromatischen Lösungsmittels zu unterziehen;
5 ) das Alkylhalogendifluoracetat (I) zu gewinnen.

**2.** Verfahren nach Anspruch 1, dadurch gekennzeichnet, daß das 1,1-Difluortetrahalogenethan der Formel (II) $CF_2BrCCl_2Br$ oder $CF_2BrCCl_2I$ ist.

**3.** Verfahren nach Anspruch 1, dadurch gekennzeichnet, daß der Alkohol ROH (III) Ethanol oder Isopropanol ist.

**4.** Verfahren nach einem der Ansprüche 1 bis 3, dadurch gekennzeichnet, daß der chemische Freie-Radikale-Bildner eine Azo-Verbindung ist.

**5.** Verfahren nach Anspruch 4, dadurch gekennzeichnet, daß die Azo-Verbindung 2,2'-Azobis(2-Methylbutyronitril) ist.

**6.** Verfahren nach Anspruch 1, dadurch gekennzeichnet, daß die geringe molare Menge p des chemischen Freie-Radikale-Bildners zwischen 10 % und 20 % der gesamten verwendeten molaren Menge p+q ist.

**7.** Verfahren nach Anspruch 1, dadurch gekennzeichnet, daß das Lösungsmittel Cyclohexan oder Toluen ist.

**8.** Verfahren nach einem der Ansprüche 1 bis 7, dadurch gekennzeichnet, daß man in Gegenwart von Luft arbeitet.

**9.** Verfahren nach Anspruch 1, dadurch gekennzeichnet, daß man in Gegenwart von Wasser arbeitet.

## Claims

1. Process for the preparation of alkyl halodifluoroacetates of formula:

$$CF_2X-\underset{\underset{O}{\|}}{C}-OR \qquad (II)$$

in which X represents a fluorine, chlorine, bromine or iodine atom and R represents a linear or branched aliphatic hydrocarbonaceous radical having a carbon number ranging from 1 to 10, characterized in that it comprises:

1) bringing a 1,1-difluorotetrahaloethane of formula:

$$CF_2XCY_2Z \qquad (II)$$

in which X has the same meaning as in the formula (I) and Y and Z, which are identical or different, represent a bromine, chlorine or iodine atom, into contact with an alcohol ROH (III), R having the same meaning as in the formula (I), according to an ROH/$CF_2XCY_2Z$ molar ratio at most equal to 30 and preferably between 5 and 20, in the presence of oxygen and of a minor molar amount p, which is at most equal to 40% of the total molar amount p+q employed, of at least one chemical initiator of free radicals;
2) heating the reaction mixture obtained in stage 1) to a temperature at least equal to 40°C and preferably of between 60°C and 80°C;
3) introducing, continuously or by successive additions, while maintaining the temperature of stage 2) and still in the presence of oxygen, a major molar amount q of at least one chemical initiator of free radicals such that the p+q/$CF_2XCY_2Z$ molar ratio is between 0.01 and 0.2 and preferably between 0.05 and 0.1;
4) cooling the reaction mixture to ambient temperature, then extracting a crude product by distillation of the said reaction mixture and then subjecting this said crude product to distillation in the presence of an aliphatic, cycloaliphatic or aromatic solvent;
5) recovering the alkyl halodifluoroacetate (I).

2. Process according to Claim 1, characterized in that the 1,1-difluorotetrahaloethane of formula (II) is $CF_2BrCCl_2Br$ or $CF_2BrCCl_2I$.

3. Process according to Claim 1, characterized in that the alcohol ROH (III) is ethanol or isopropanol.

4. Process according to one of Claims 1 to 3, characterized in that the chemical initiator of free radicals is an azo compound.

5. Process according to Claim 4, characterized in that the azo compound is 2,2'-azobis(2-methylbutyronitrile).

6. Process according to Claim 1, characterized in that the minor molar amount p of chemical initiator of free radicals is between 10% and 20% of the total molar amount p+q employed.

7. Process according to Claim 1, characterized in that the solvent is cyclohexane or toluene.

8. Process according to one of Claims 1 to 7, characterized in that it is carried out in the presence of air.

9. Process according to Claim 1, characterized in that it is carried out in the presence of water.